# EUROPEAN PATENT APPLICATION

(11) **EP 3 756 670 A1**
(43) Date of publication of application: **30.12.2020**
(21) Application number: 19756715.9
(22) Date of filing: 20.02.2019
(51) Int. Cl.: A61K 31/4965, A61K 9/14, A61K 9/16, A61K 9/20, A61K 9/48, A61K 47/26, A61K 47/36, A61P 7/00, A61P 7/02, A61P 9/08, A61P 11/08, A61P 43/00

(54) **GRANULAR COMPOSITION, METHOD FOR PRODUCING GRANULAR COMPOSITION, AND METHOD FOR IMPROVING ELUTION PROPERTY OF GRANULAR COMPOSITION**

(30) Priority: 21.02.2018 JP 2018029093
(71) Applicant: Nippon Shinyaku Co., Ltd., Kyoto-shi Kyoto 601-8550 (JP)
(72) Inventor: TANAKA Toshinori, Kyoto-shi, Kyoto 601-8550 (JP); YAMADA Rie, Kyoto-shi, Kyoto 601-8550 (JP)
(74) Representative: Vossius & Partner Patentanwälte Rechtsanwälte mbB
(86) International application number: PCT/JP2019/006317
(87) International publication number: WO 2019/163822

(57) **Abstract**

A production method for a granular composition in which the dissolution property of 2-{4-[N-(5,6-diphenylpyradin-2-yl)-N-isopropylamino]butyloxy}-N-(methylsulfonyl)acetamide is improved is provided.

The production method for a granular composition includes a step of compression molding a mixture obtained by mixing 2-{4-[N-(5,6-diphenylpyradin-2-yl)-N-isopropylamino]butyloxy}-N-(methylsulfonyl)acetamide and at least one or more excipients selected from the group consisting of a sugar alcohol, a starch, and a saccharide, thereby obtaining a compression molded material.

## Description

### Technical Field

The present invention relates to a granular composition containing 2-{4-[N-(5,6-diphenylpyradin-2-yl)-N-isopropylamino]butyloxy}-N-(methylsulfonyl)acetamide (hereinafter referred to as "Compound (I)"). Further, the present invention relates to a production method for a granular composition containing Compound (I). Further, the present invention relates to a dissolution property improvement method for improving the dissolution property of Compound (I) in a granular composition containing Compound (I).

### Background Art

It is known that Compound (I) represented by the following structural formula has an excellent prostaglandin I₂ (also referred to as PGI₂) receptor agonistic effect and shows various medicinal effects such as a platelet aggregation inhibitory effect, a vasodilating effect, a bronchial smooth muscle dilating effect, a lipid deposition inhibitory effect, and a leukocyte activation inhibitory effect (for example, Patent Document 1). Further, Compound (I) is prescribed as a tablet.

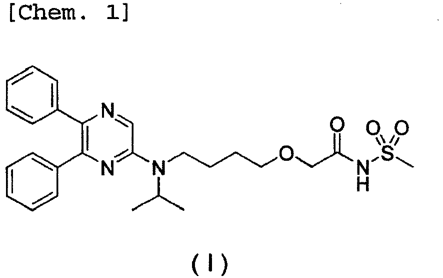

### Prior Art Documents

### Patent Document

[Patent Document 1] WO 2002/088084

### Non-Patent Documents

[Non-Patent Document 1] Hepatology, 2007, Vol. 45, No. 1, pp. 159-169
[Non-Patent Document 2] Folia Pharmacologica Japonica, Vol. 117, No. 2, pp. 123-130, 2001, Abstract
[Non-Patent Document 3] International Angiology, 29, Suppl. 1 to No. 2, pp. 49-54, 2010
[Non-Patent Document 4] Jpn. J. Clin. Immunol., 16(5), 409-414, 1993
[Non-Patent Document 5] Jpn. J. Thromb. Hemost., 1:2, pp. 94-105, 1990, Abstract
[Non-Patent Document 6] J. Rheumatol., 2009, 36(10), 2244-2249
[Non-Patent Document 7] Japan J. Pharmacol., 43, pp. 81-90, 1987
[Non-Patent Document 8] New Engl. J. Med., 2015, 24, 2522-2533
[Non-Patent Document 9] CHEST 2003, 123, 1583-1588
[Non-Patent Document 10] Br. Heart J., 53, pp. 173-179, 1985
[Non-Patent Document 11] The Lancet, 1, 4880, pt 1, pp. 569-572, 1981
[Non-Patent Document 12] Eur. J. Pharmacol., 449, pp. 167-176, 2002
[Non-Patent Document 13] The Journal of Clinical Investigation, 117, pp. 464-472, 2007
[Non-Patent Document 14] Am. J. Physiol. Lung Cell Mol. Physiol., 296: L648-L656, 2009

### Summary of the Invention

### Problems to be solved by the Invention

It is generally difficult for children and the elderly having a poor swallowing ability to take a tablet. As a tablet which is easily swallowed, an orally disintegrating tablet or a chewable tablet has been developed but cannot necessarily be said to be a tablet which is easily taken for the elderly with little salivation.

On the other hand, a granular preparation (granular composition) such as a powder, a fine granule, a granule, a granular tablet, or a dry syrup is easily taken also by the elderly, and therefore, medication compliance is improved, and also the degree of freedom in changing the dose is increased, and therefore, such a preparation is very useful.

Further, in a case where a preparation is produced, a formulation technique for increasing the dissolution property of a medicinal component is generally used. In general, the dissolution property of a medicinal component from a tablet depends on a time until the tablet disintegrates into a granule or a powder. Therefore, in a case of a tablet, prompt dissolution of a medicinal component cannot be expected as compared with a case of a granule or a powder.

In view of this, a formulation of a granular composition containing Compound (I) has been desired. A granular composition such as a granule is generally a granulated material and is generally prepared by a fluidized bed granulation method or the like. However, in the process of studying the formulation of a granule containing Compound (I), it was revealed that the dissolution property of Compound (I) is low in a granule obtained by a fluidized bed granulation method. That is, it was revealed that in a granular composition containing Compound (I), only adhering an excipient or the like to Compound (I) leads to the dissolution of Compound (I) being slow and the dissolution property being low.

An object of the present invention is to provide a production method for a granular composition capable of improving the dissolution property of Compound (I). Further, an object of the present invention is to provide a dissolution property improvement method capable of improving the dissolution property of Compound (I) in a granular composition. Further, an object of the present invention is to provide a granular composition capable of improving the dissolution property of Compound (I).

### Means for Solving the Problems

As a result of intensive studies for solving the above problems, the present inventors found that the dissolution property of Compound (I) is improved by mixing Compound (I) and at least one or more excipients selected from the group consisting of a sugar alcohol, a starch, and a saccharide, followed by compression molding in the production of a granular composition, and thus completed the present invention.

The present invention is a production method for a granular composition containing Compound (I), which includes a compression molding step of compression molding a mixture obtained by mixing Compound (I) and at least one or more excipients selected from the group consisting of a sugar alcohol, a starch, and a saccharide, thereby obtaining a compression molded material.

Further, in the present invention, it is preferred that in the production method for a granular composition having the above constitution, the dissolution property of Compound (I) in the granular composition is higher than the dissolution property of Compound (I) in the mixture before the compression molding step.

Further, in the present invention, it is preferred that in the production method for a granular composition having the above constitution, the porosity of the granular composition is 45% or less.

Further, in the present invention, it is preferred that in the production method for a granular composition having the above constitution, the granule size of the granular composition is smaller than 5 mm.

Further, in the present invention, it is preferred that in the production method for a granular composition having the above constitution, the compression molding step is performed by any one of a roller compression method, a tableting compression method, a briquetting method, a slugging method, and an extrusion granulation method.

Further, in the present invention, it is preferred that in the production method for a granular composition having the above constitution, in the compression molding step, the extrusion granulation method is performed using an extruder which extrudes the mixture through a hole portion, and the diameter of the hole portion is from 0.2 mm to 0.5 mm.

Further, in the present invention, it is preferred that in the production method for a granular composition having the above constitution, a crushing step of crushing the compression molded material is further included.

Further, in the present invention, it is preferred that in the production method for a granular composition having the above constitution, the granular composition is a granule, a powder, a filler of a capsule, a granular tablet, a dry syrup, or a fine granule.

Further, the present invention is a dissolution property improvement method for improving the dissolution property of Compound (I) in a granular composition containing Compound (I), which includes a compression molding step of compression molding a mixture obtained by mixing Compound (I) and at least one or more excipients selected from the group consisting of a sugar alcohol, a starch, and a saccharide, thereby obtaining a compression molded material.

Further, in the present invention, it is preferred that in the dissolution property improvement method having the above constitution, the dissolution property of Compound (I) in the granular composition is higher than the dissolution property of Compound (I) in the mixture before the compression molding step.

Further, in the present invention, it is preferred that in the dissolution property improvement method having the above constitution, the porosity of the granular composition is 45% or less.

Further, in the present invention, it is preferred that in the dissolution property improvement method having the above constitution, the granule size of the granular composition is smaller than 5 mm.

Further, in the present invention, it is preferred that in the dissolution property improvement method having the above constitution, the compression molding step is performed by any one of a roller compression method, a tableting compression method, a briquetting method, a slugging method, and an extrusion granulation method.

Further, in the present invention, it is preferred that in the dissolution property improvement method having the above constitution, in the compression molding step, the extrusion granulation method is performed using an extruder which extrudes the mixture through a hole portion, and the diameter of the hole portion is from 0.2 mm to 0.5 mm.

Further, in the present invention, it is preferred that in the dissolution property improvement method having the above constitution, a crushing step of crushing the compression molded material is further included.

Further, in the present invention, it is preferred that in the dissolution property improvement method having the above constitution, the granular composition is a granule, a powder, a filler of a capsule, a granular tablet, a dry syrup, or a fine granule.

A granular composition of the present invention is in a state where Compound (I) and at least one or more excipients selected from the group consisting of a sugar alcohol, a starch, and a saccharide are mixed, and has a porosity of 45% or less.

Further, in the present invention, it is preferred that in the granular composition having the above constitution, the granular composition has a granule size smaller than 5 mm.

### [Effects of the Invention]

According to the production method for a granular composition of the present invention, a granular composition in which the dissolution property of Compound (I) is improved can be obtained. Further, according to the dissolution property improvement method of the present invention, the dissolution property of Compound (I) in the granular composition can be improved. Further, according to the granular composition of the present invention, the dissolution property of Compound (I) can be improved.

### Brief Description of the Drawings

[FIG. 1] FIG. 1 is a powder X-ray diffraction spectrum chart of a Form-I crystal of Compound (I) contained in a granular composition of an embodiment of the present invention. The vertical axis represents a peak intensity (unit: cps) and the horizontal axis represents a diffraction angle 2θ (unit: °).
[FIG. 2] FIG. 2 is a powder X-ray diffraction spectrum chart of a Form-II crystal of Compound (I) contained in a granular composition of an embodiment of the present invention. The vertical axis represents a peak intensity (unit: cps) and the horizontal axis represents a diffraction angle 2θ (unit: °).
[FIG. 3] FIG. 3 is a powder X-ray diffraction spectrum chart of a Form-III crystal of Compound (I) contained in a granular composition of an embodiment of the present invention. The vertical axis represents a peak intensity (unit: cps) and the horizontal axis represents a diffraction angle 2θ (unit: °).
[FIG. 4] FIG. 4 is a process chart showing a production step of a granular composition of an embodiment of the present invention.
[FIG. 5] FIG. 5 is a view showing the time course of the dissolution rate of Compound (I) in Example 1 and Comparative Example 1. The vertical axis represents the dissolution rate (unit: %) and the horizontal axis represents a time (unit: min).
[FIG. 6] FIG. 6 is a view showing the time course of the dissolution rate of Compound (I) in Example 2 and Comparative Example 2. The vertical axis represents the dissolution rate (unit: %) and the horizontal axis represents a time (unit: min).
[FIG. 7] FIG. 7 is a view showing the time course of the dissolution rate of Compound (I) in Example 3 and Comparative Example 3. The vertical axis represents the dissolution rate (unit: %) and the horizontal axis represents a time (unit: min).
[FIG. 8] FIG. 8 is a view showing the time course of the dissolution rate of Compound (I) in Example 4 and Comparative Example 4. The vertical axis represents the dissolution rate (unit: %) and the horizontal axis represents a time (unit: min).
[FIG. 9] FIG. 9 is a view showing the time course of the dissolution rate of Compound (I) in Example 5 and Comparative Example 5. The vertical axis represents the dissolution rate (unit: %) and the horizontal axis represents a time (unit: min).
[FIG. 10] FIG. 10 is a view showing the time course of the dissolution rate of Compound (I) in Examples 6 to 8 and Comparative Example 6. The vertical axis represents the dissolution rate (unit: %) and the horizontal axis represents a time (unit: min).
[FIG. 11] FIG. 11 is a view showing the time course of the dissolution rate of Compound (I) in Comparative Examples 7 and 8. The vertical axis represents the dissolution rate (unit: %) and the horizontal axis represents a time (unit: min).

### Mode for Carrying Out the Invention

Hereinafter, a granular composition of an embodiment of the present invention will be described. The "granular composition" as used herein means a material obtained by processing a powder raw material into a granular form which is larger than the powder raw material through the below-mentioned mixing step and compression molding step.

### <1. Constitution of Granular Composition>

The granular composition of this embodiment includes, for example, a granule, a powder, a fine granule, a granular tablet, a dry syrup, and the like. Further, the granular composition can be used, for example, as an oral solid preparation for direct oral administration. Further, the granular composition can also be used, for example, as a suspension obtained by dispersing the composition in water, a syrup, or the like. Further, the granular composition can also be used by being filled in a capsule. That is, the granular composition can be utilized as a filler of a capsule.

The granular composition contains Compound (I) and an excipient. For example, Compound (I) can be easily produced according to the method described in Patent Document 1. Further, in Compound (I), there exist the following three forms of crystals (a Form-I crystal, a Form-II crystal, and a Form-III crystal).

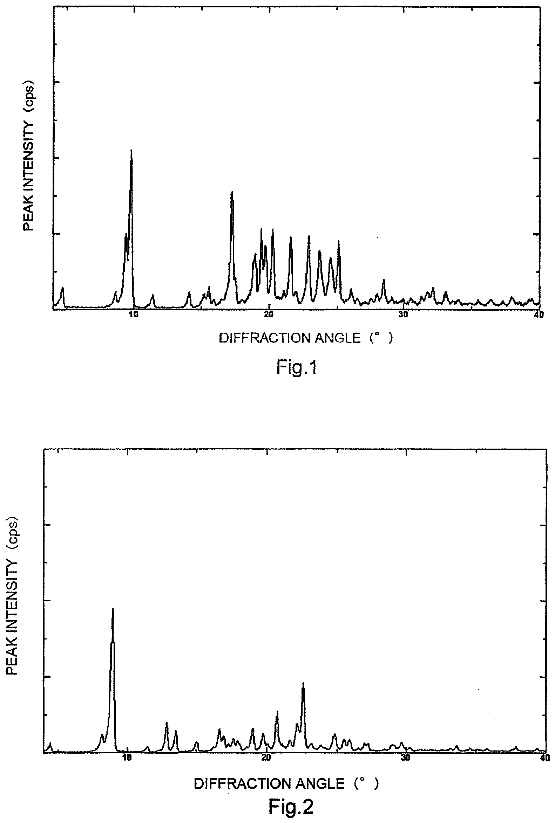

FIGS. 1 to 3 are the powder X-ray diffraction spectrum charts (powder X-ray diffraction diagrams) of the Form-I crystal, the Form-II crystal, and the Form-III crystal, respectively. In each drawing, the vertical axis represents a peak intensity (unit: cps) and the horizontal axis represents a diffraction angle 2θ (unit: °). The powder X-ray diffraction spectrum was measured using an X-ray diffractometer (RINT-Ultima III, manufactured by Rigaku Corporation). At this time, the target was Cu, the voltage was set to 40 kV, the current was set to 40 mA, and the scan speed was set to 4°/min.
(1) The powder X-ray diffraction diagram of the Form-I crystal is obtained using a Cu-Kα radiation (λ=1.54 Å), and the Form-I crystal shows diffraction peaks at the following diffraction angles (2θ): 9.4°, 9.8°, 17.2°, and 19.4° in the powder X-ray diffraction spectrum of Compound (I) .
(2) The powder X-ray diffraction diagram of the Form-II crystal is obtained using a Cu-Kα radiation (λ=1.54 Å), and the Form-II crystal shows diffraction peaks at the following diffraction angles (2θ):9.0°, 12.9°, 20.7°, and 22.6° in the powder X-ray diffraction spectrum of Compound (I) .
(3) The powder X-ray diffraction diagram of the Form-III crystal is obtained using a Cu-Kα radiation (λ=1.54 Å), and the Form-III crystal shows diffraction peaks at the following diffraction angles (2θ) : 9.3°, 9.7°, 16.8°, 20.6°, and 23.5° in the powder X-ray diffraction spectrum of Compound (I).

Compound (I) contained in the granular composition may be any of the above-mentioned Form-I, Form-II, and Form-III crystals, or may be a mixture of these crystals, or may be amorphous. As the crystal of Compound (I), the Form-I crystal is preferred.

The excipient contained in the granular composition may be at least one or more excipients selected from the group consisting of a sugar alcohol, a starch, and a saccharide. Incidentally, the sugar alcohol, the starch, and the saccharide are contained preferably in an amount of 1 to 30000 weights, more preferably in an amount of 100 to 6000 weights, further more preferably in an amount of 300 to 4000 weights with respect to 1 weight of Compound (I).

As an example of the sugar alcohol, D-mannitol, erythritol, xylitol, D-sorbitol, isomalt, maltitol, lactitol, and the like can be exemplified. D-Mannitol, erythritol, xylitol, D-sorbitol, and isomalt are preferred, and D-mannitol, erythritol, and isomalt are more preferred.

As an example of the starch, cornstarch, potato starch, rice starch, wheat starch, and the like can be exemplified. Cornstarch and potato starch are preferred, and cornstarch is more preferred.

As an example of the saccharide, maltose, trehalose, lactose, glucose, fructose, sucrose, and the like can be exemplified. Maltose, trehalose, glucose, and lactose are preferred, and glucose and lactose are more preferred.

As will be described in detail later, the granular composition is in a state where a mixture of Compound (I) and the excipient is compression molded. According to this, the dissolution property of Compound (I) in the granular composition can be improved. Further, when the porosity of the granular composition is 45% or less, the dissolution property of Compound (I) can be further improved, and therefore, such a configuration is preferred. Incidentally, the porosity will be described in detail later.

Further, when the granule size of the granular composition is smaller than 5 mm, the composition is easily taken by a person who takes the composition, and also the degree of freedom in changing the dose is increased, and therefore, such a configuration is preferred. When the granule size of the granular composition is 3 mm or less, the composition is more easily taken by a person who takes the composition, and also the degree of freedom in changing the dose is further increased, and therefore, such a configuration is more preferred. Here, the "granule size" means an "average granule size" and is measured by a microscopic method (visual observation method) or an image analysis method.

The granular composition may also contain various types of pharmaceutical additives in addition to the excipient. The pharmaceutical additives are not particularly limited as long as they are pharmaceutically acceptable and also pharmacologically acceptable, and for example, a binder, a disintegrant, a lubricant, a fluidizing agent, a coloring agent, a coating agent, a taste masking agent, a foaming agent, a sweetener, a flavoring agent, an antioxidant, a surfactant, a plasticizer, a sugar coating agent, and the like can be exemplified. These pharmaceutical additives may be used alone or two or more types may be used in combination.

When the granular composition is coated with a coating agent or a sugar coating agent by a known method, it is possible to try to improve the aesthetic appearance of the granular composition or ensure the discriminability thereof, and therefore, such a configuration is preferred. Further, when a coloring agent is incorporated in the granular composition, it is possible to try to improve the light stability of the granular composition or ensure the discriminability thereof, and therefore, such a configuration is preferred. Further, when a taste masking agent or a flavoring agent is incorporated in the granular composition, it is possible to easily improve the flavor of the granular composition, and therefore, such a configuration is preferred.

As the binder, for example, gelatin, pullulan, hydroxypropyl cellulose, methyl cellulose, hypromellose, polyvinylpyrrolidone, macrogol, gum Arabic, dextran, polyvinyl alcohol, pregelatinized starch, and the like can be exemplified.

As the disintegrant, for example, carmellose, carmellose calcium, carmellose sodium, croscarmellose sodium, sodium starch glycolate, crospovidone, low-substituted hydroxypropyl cellulose, partially pregelatinized starch, crystalline cellulose, cornstarch, and the like can be exemplified.

As the lubricant, for example, stearic acid, magnesium stearate, calcium stearate, sodium stearyl fumarate, talc, waxes, DL-leucine, sodium lauryl sulfate, magnesium lauryl sulfate, macrogol, light anhydrous silicic acid, and the like can be exemplified.

As the fluidizing agent, for example, light anhydrous silicic acid, hydrous silicon dioxide, synthetic aluminum silicate, magnesium aluminometasilicate, calcium silicate, and the like can be exemplified.

As the coloring agent, for example, titanium oxide, talc, iron sesquioxide, yellow iron sesquioxide, Food Yellow No. 4, Food Yellow No. 4 Aluminum Lake, and the like can be exemplified.

As the coating agent, hypromellose, hydroxypropyl cellulose, polyvinyl alcohol, ethyl cellulose, an ethyl acrylate-methyl methacrylate copolymer, methacrylic acid copolymer LD, hypromellose acetate succinate, and the like can be exemplified.

As the taste masking agent, for example, fructose, xylitol, glucose, DL-malic acid, and the like can be exemplified.

As the foaming agent, for example, sodium hydrogen carbonate, dried sodium carbonate, calcium carbonate, and the like can be exemplified.

As the sweetener, for example, aspartame, acesulfame potassium, sucralose, thaumatin, fructose, glucose, Glycyrrhiza, xylitol, and the like can be exemplified.

As the flavoring agent, for example, L-menthol, peppermint, and the like can be exemplified.

As the antioxidant, for example, sodium nitrite, ascorbic acid, natural vitamin E, tocopherol, and the like can be exemplified.

As the surfactant, for example, sodium lauryl sulfate, sorbitan monooleate, squalane, and the like can be exemplified.

As the plasticizer, for example, triethyl citrate, propylene glycol, macrogol, and the like can be exemplified.

As the sugar coating agent, for example, sucrose, precipitated calcium carbonate, gum Arabic, polyvinyl alcohol, kaolin, titanium oxide, macrogol, stearic acid, ethyl cellulose, and the like can be exemplified.

Compound (I) has an excellent PGI₂ receptor agonistic effect and is useful as a preventive agent or a therapeutic agent for a PGI₂-related disease, for example, transient ischemic attack (TIA), diabetic neuropathy (see, for example, Non-Patent Document 1), diabetic gangrene (see, for example, Non-Patent Document 1), a peripheral circulatory disturbance (for example, chronic arteriosclerosis or chronic arterial occlusion (see, for example, Non-Patent Document 2)), intermittent claudication (see, for example, Non-Patent Document 3), peripheral embolism (see, for example, Non-Patent Document 5), Raynaud's disease (see, for example, Non-Patent Document 4), a connective tissue disease (for example, systemic lupus erythematosus or scleroderma) (see, for example, Non-Patent Document 6), a mixed connective tissue disease, a vasculitis syndrome, reocclusion/restenosis after percutaneous transluminal coronary angioplasty (PTCA), arteriosclerosis, thrombosis (for example, acute-phase cerebral thrombosis or pulmonary embolism) (see, for example, Non-Patent Document 5 or Non-Patent Document 7), hypertension, pulmonary hypertension such as pulmonary arterial hypertension or chronic thromboembolic pulmonary hypertension (see, for example, Non-Patent Document 8 or Non-Patent Document 9), an ischemic disease (for example, cerebral infarction or myocardial infarction (see, for example, Non-Patent Document 10)), angina pectoris (for example, stable angina pectoris or unstable angina pectoris) (see, for example, Non-Patent Document 11), glomerulonephritis (see, for example, Non-Patent Document 12), diabetic nephropathy (see, for example, Non-Patent Document 1), chronic renal failure, allergy, bronchial asthma (see, for example, Non-Patent Document 13), ulcer, pressure ulcer (bedsore), restenosis after coronary intervention such as atherectomy or stent implantation, thrombocytopenia by dialysis, a disease related to fibrogenesis in an organ or a tissue [for example, a renal disease (for example, tubulointerstitial nephritis), a respiratory disease (for example, interstitial pneumonia (pulmonary fibrosis), a chronic obstructive pulmonary disease (see, for example, Non-Patent Document 14), or the like), a digestive disease (for example, hepatocirrhosis, viral hepatitis, chronic pancreatitis, or scirrhous gastric cancer), a cardiovascular disease (for example, myocardial fibrosis), a bone or articular disease (for example, bone marrow fibrosis or rheumatoid arthritis), a skin disease (for example, postoperative cicatrix, burn cicatrix, keloid, or hypertrophic cicatrix), an obstetric disease (for example, uterine fibroid), a urinary disease (for example, prostatic hypertrophy), other diseases (for example, Alzheimer's disease, sclerosing peritonitis, type I diabetes, or postoperative organ adhesion)], erectile dysfunction (for example, diabetic erectile dysfunction, psychogenic erectile dysfunction, psychotic erectile dysfunction, erectile dysfunction due to chronic renal failure, erectile dysfunction after pelvic operation for resection of the prostate, or vascular erectile dysfunction associated with aging or arteriosclerosis), an inflammatory bowel disease (for example, ulcerative colitis, Crohn's disease, intestinal tuberculosis, ischemic colitis, or intestinal ulcer associated with Behcet disease), gastritis, gastric ulcer, an ischemic eye disease (for example, retinal artery occlusion, retinal vein occlusion, or ischemic optic neuropathy), sudden hearing loss, avascular necrosis of bone, an intestinal damage associated with administration of a non-steroidal anti-inflammatory agent (NSAID) (for example, diclofenac, meloxicam, oxaprozin, nabumetone, indomethacin, ibuprofen, ketoprofen, naproxen, or celecoxib) (while there is no particular limitation as long as it is a damage occurring in, for example, the duodenum, small intestine, or large intestine, for example, a mucosal damage such as erosion or ulcer occurring in the duodenum, small intestine, or large intestine), or a symptom (for example, paralysis, dullness in sensory perception, pain, numbness, or a decrease in walking ability) associated with spinal canal stenosis (for example, cervical spinal canal stenosis, thoracic spinal canal stenosis, lumbar spinal canal stenosis, coexisting cervical and lumbar spinal stenosis, or sacral spinal stenosis). In addition, the granular composition of the present invention is also useful as an accelerating agent for gene therapy or angiogenic therapy such as autologous bone marrow transplantation, or an accelerating agent for angiogenesis in peripheral revascularization or angiogenic therapy.

### <2. Production Method for Granular Composition and Dissolution Property Improvement Method for Compound (I) of this Embodiment>

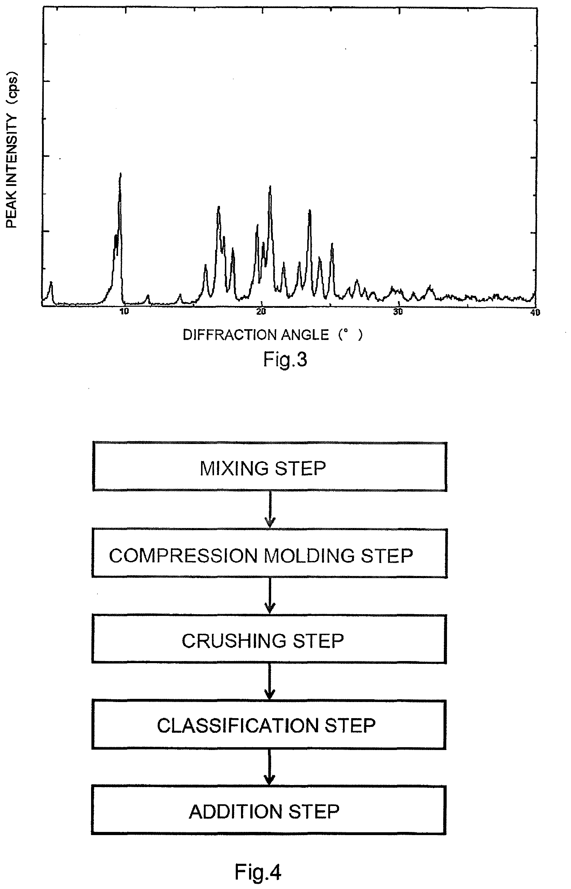

Next, a production method for the granular composition will be described. FIG. 4 is a process chart showing a production step of the granular composition. The production step includes a mixing step, a compression molding step, a crushing step, a classification step, and an addition step. Incidentally, a dissolution property improvement method for improving the dissolution property of Compound (I) in the granular composition is also performed in the same manner as the production method.

### <2-1. Mixing Step>

In the mixing step, Compound (I) in the form of a powder, and at least one or more excipients in the form of a powder selected from the group consisting of a sugar alcohol, a starch, and a saccharide are uniformly mixed, whereby a mixture is obtained. Incidentally, the "mixing" also includes a case where so-called "granulation" is performed such that Compound (I) and the excipient are uniformly mixed and small particles are grown to large particles by mutually adhering and aggregating a plurality of small particles.

The mixing step is performed using a mixer. The mixer is not particularly limited, and for example, a container rotary-type mixer, a mechanical stirring-type mixer, an airflow-type mixer, a kneading-type mixer, or the like can be used. Further, the mixing step may be performed using a granulator as the mixer. The granulator is not particularly limited, and for example, a fluidized bed granulator, a stirring granulator, a rotary granulator, or the like can be used.

### <2-2. Compression Molding Step>

In the compression molding step after the mixing step, the mixture prepared in the mixing step is compression molded, whereby a compression molded material is obtained. At this time, it is preferred that the porosity of the compression molded material is 45% or less. The compression molding step is performed using a compression molding machine. The compression molding method is not particularly limited, and for example, a roller compression method, a tableting compression method, a briquetting method, a slugging method, or an extrusion granulation method is preferred.

In the roller compression method (roller compacting method), a roller compactor is used as the compression molding machine. The roller compactor has two rolls in which a rotation axis is horizontally disposed. The two rolls are disposed facing each other in a direction orthogonal to the rotation axis. A predetermined gap is provided between the two rolls, and the two rolls rotate in mutually opposite directions.

The mixture obtained in the mixing step is supplied to the gap between the rotating two rolls, and the mixture is compression molded by applying a pressure thereto with the two rolls. In this manner, the compression molding step is performed by the roller compression method, whereby a sheet-shaped (thin plate-shaped) or flake-shaped compression molded material is formed. Incidentally, the surface of the roll may be smooth or may have a plurality of fine irregularities. When a plurality of fine irregularities are provided on the surface of the roll, the mixture is easily retained on the roll, so that the compression efficiency can be improved, and therefore, such a configuration is preferred.

At this time, the magnitude of the pressure applied to the mixture is not particularly limited as long as the pressure has such a magnitude that the dissolution property of Compound (I) can be improved, and the pressure is preferably 0.5 N/mm² or more, more preferably from 0.5 to 25 N/mm², and further more preferably from 0.5 to 10 N/mm².

In the tableting compression method (tableting method), a tableting machine is used as the compression molding machine. As the tableting machine, for example, a single-shot type tableting machine, a rotary-type tableting machine, or the like can be used. The tableting machine has a cylindrical mortar and a pair of upper and lower metal rods (an upper punch and a lower punch). In the compression molding step, the upper punch and the lower punch sandwich the mixture filled in the mortar in the vertical direction and perform compression molding. In this manner, the compression molding step is performed by the tableting compression method, whereby a disk-shaped compression molded material is formed.

At this time, the magnitude of the pressure applied to the mixture is not particularly limited as long as the pressure has such a magnitude that the dissolution property of Compound (I) can be improved, and the pressure is preferably 10 N/mm² or more. Further, the pressure applied to the mixture is more preferably from 10 to 1500 N/mm², and further more preferably from 10 to 700 N/mm².

In the briquetting method, a briquetting machine is used as the compression molding machine. The briquetting machine has two rolls in which a rotation axis is horizontally disposed. The two rolls are disposed facing each other in a direction orthogonal to the rotation axis. A predetermined gap is provided between the two rolls, and the two rolls rotate in mutually opposite directions. On the surface of the roll, a plurality of pockets are provided in a recessed manner and are arranged in the rotational direction of the roll. Incidentally, the pocket is a matrix of the briquette, and it is preferred that the volume of the pocket is from about 0.3 cm³ to about 200 cm³.

In the compression molding step, the mixture prepared in the mixing step is supplied to the gap between the rotating two rolls, and the mixture is compression molded by applying a pressure thereto with the two rolls. In this manner, the compression molding step is performed by the briquetting method, whereby a briquette (compression molded material) is formed.

At this time, the magnitude of the pressure applied to the mixture is not particularly limited as long as the pressure has such a magnitude that the dissolution property of Compound (I) can be improved, and the pressure is preferably 10 N/mm² or more. Further, the pressure applied to the mixture is more preferably from 10 to 1500 N/mm², and further more preferably from 10 to 700 N/mm².

In the extrusion granulation method, an extrusion granulator is used as the compression molding machine. The extrusion granulator has a storage chamber in which the mixture prepared in the mixing step is stored and a plurality of circular hole portions are opened, and a pressing portion which presses the mixture in the storage chamber toward the plurality of hole portions. The extrusion system of the extrusion granulator includes a screw extrusion system, a plunger extrusion system, a roller extrusion system, and the like. The pressing portion corresponds to a screw, a plunger, and a roller, respectively. Further, the hole portion is, for example, a die hole or a hole of a screen (porous plate). In a case of the screw extrusion system, the production efficiency of the granular composition can be easily improved, and therefore, such a system is preferred.

In a case where the extrusion granulation method is used, in the mixing step, a solvent is added to Compound (I) and the excipient, followed by kneading. By doing this, a kneaded material (mixture) is obtained. As the solvent, for example, water or ethanol or various binder solutions (aqueous solution or aqueous solution containing ethanol) and the like can be exemplified. In the compression molding step, the kneaded material is stored in the storage chamber of the extrusion granulator, and the kneaded material is extruded outside the extrusion granulator from the hole portions by the pressing portion of the extrusion granulator. By doing this, a columnar compression molded material is obtained.

Incidentally, the diameter of the hole portion of the extrusion granulator is preferably 0.5 mm or less, and more preferably from 0.2 to 0.5 mm. The cross-sectional area orthogonal to the extrusion direction of the storage chamber of the extrusion granulator is generally sufficiently larger than the area of the hole portion, and therefore, when the diameter of the hole portion is set to 0.5 mm or less, a pressure can be more sufficiently applied to the kneaded material. Incidentally, in a case where an extrusion granulator (for example, a twin-screw type, or the like) which can apply a sufficiently large pressure to the kneaded material due to the configuration of the extrusion portion is used, the diameter of the hole portion of the extrusion granulator may be larger than 0.5 mm.

The slugging method is a method in which a pressure is applied to the mixture prepared in the mixing step in a dry state as such, whereby a columnar powder compression molded block (slug or compression molded material) is formed. The size of the powder compression molded block is not particularly limited, and the diameter of the powder compression molded block can be set to, for example, about 20 mm.

As described above, by performing the compression molding step using a roller compression method, a tableting compression method, a briquetting method, a slugging method, or an extrusion granulation method, the compression molded material can be easily formed.

### <2-3. Crushing Step>

In the crushing step after the compression molding step, the compression molded material is crushed using a crusher or the like. By the crushing step, a granular crushed material is formed from the compression molded material. In the following description, the "granular crushed material" is sometimes referred to as "formed granular material".

Incidentally, after the compression molding step and before the crushing step, a disintegration step of disintegrating the compression molded material using a disintegrator may be performed. By doing this, in the crushing step, the compression molded material can be stably crushed.

### <2-4. Classification Step>

In the classification step after the crushing step, the crushed material is classified using an airflow-type classifier, a sieve, or the like. By doing this, the formed granular material having a desired granule size can be easily obtained. Incidentally, the crushed material removed in the classification step due to insufficient crushing may be crushed again in the crushing step.

### <2-5. Addition Step>

In the addition step after the classification step, the formed granular material classified in the classification step and a pharmaceutical additive are mixed. The mixing method in the addition step is the same as the mixing method in the above-mentioned mixing step. By the addition step, the pharmaceutical additive is added to the formed granular material.

By the above-mentioned production step, the granular composition is formed. According to the production method of this embodiment, the compression molding step is included. Accordingly, the dissolution of Compound (I) is quickened, and the granular composition capable of improving the dissolution property of Compound (I) can be easily formed. Further, according to the dissolution property improvement method of this embodiment, the compression molding step is included. Accordingly, the dissolution of Compound (I) is quickened, and the dissolution property of Compound (I) in the granular composition can be improved.

Further, the dissolution property of Compound (I) in the granular composition is higher than the dissolution property of Compound (I) in the mixture before the compression molding step.

Incidentally, in this embodiment, the production method for the granular composition may only include the compression molding step, and there is no particular limitation on the other steps. For example, a general method described in a publication such as Powder Technology and Pharmaceutical Processes (D. Chulia, et al., Elsevier Science Pub. Co. (December 1, 1993)) may be used.

Further, in the mixing step, mixing may be performed by further adding a pharmaceutical additive other than the excipient in addition to the excipient.

Further, in the compression molding step, the pressure applied to the mixture may be gradually increased with the lapse of time. Further, the pressure applied in the former part of the compression molding step may be set larger than the pressure applied in the latter part of the compression molding step. According to this, damage such as cracking of the compression molded material can be prevented, and the compression molded material can be stably formed.

Hereinafter, the present invention will be more specifically described with reference to Examples, however, the present invention is not limited to these Examples.

**[Table 1]**

| | | Excipient |
|---|---|---|
| Example 1 | Comparative Example 1 | D-mannitol |
| Example 2 | Comparative Example 2 | Erythritol |
| Example 3 | Comparative Example 3 | Isomalt |
| Example 4 | Comparative Example 4 | Cornstarch |
| Example 5 | Comparative Example 5 | Lactose hydrate |

**[Table 2]**

| | Compression molding method |
|---|---|
| Example 6 | Roller compression method |
| Example 7 | Tableting compression method |
| Example 8 | Extrusion granulation method |
| Comparative Example 6 | Without compression molding |

Table 1 shows the excipient contained in each of the granular compositions of Examples 1 to 5, and Comparative Examples 1 to 5. Table 2 shows the compression molding method in the compression molding step of the production method for each of the granular compositions of Examples 6 to 8.

### Example 1

A granular composition of Example 1 was prepared using a slugging method. In the mixing step, 3 mg of Compound (I) and 297 mg of D-mannitol (Mannit P, manufactured by Mitsubishi Shoji Foodtech Co., Ltd.) were mixed, whereby 300 mg of a mixture was obtained. Subsequently, in the compression molding step, a pressure of 130.1 N/mm² was applied to the mixture using a precision universal testing machine (AG-X, manufactured by Shimadzu Corporation), whereby a compression molded material was obtained. In the crushing step, the compression molded material was crushed, and a 20 mg portion of the formed granular material having passed through a sieve with a mesh size of 1700 µm in the classification step was used as the granular composition (granule) of Example 1. At this time, the compression molded material was crushed such that all crushed material passed through the sieve.

### Example 2

In a granular composition of Example 2, erythritol (erythritol 50M, manufactured by B Food Science Co., Ltd.) was used as the excipient. The preparation was performed in the same manner as in Example 1 except this.

### Example 3

In a granular composition of Example 3, isomalt (galenIQ 720, manufactured by BENEO Palatinit GmbH) was used as the excipient. The preparation was performed in the same manner as in Example 1 except this.

### Example 4

In a granular composition of Example 4, cornstarch (Nisshoku Cornstarch W, manufactured by Nihon Shokuhin Kako Co., Ltd.) was used as the excipient. The preparation was performed in the same manner as in Example 1 except this.

### Example 5

In a granular composition of Example 5, lactose hydrate (Pharmatose (registered trademark) 200M, manufactured by DFE Pharma) was used as the excipient. The preparation was performed in the same manner as in Example 1 except this.

### Example 6

A granular composition of Example 6 was prepared using a roller compression method. In the mixing step, 0.2 mg of Compound (I), 900 mg of D-mannitol (Mannit P, manufactured by Mitsubishi Shoji Foodtech Co., Ltd.), and 99.8 mg of cornstarch (Nisshoku Cornstarch W, manufactured by Nihon Shokuhin Kako Co., Ltd.) were mixed, whereby 1000 mg of a mixture was obtained. Subsequently, in the compression molding step, a pressure of 10 N/mm² was applied to the mixture using a roller compactor (TF-MINI, manufactured by Freund Corporation), whereby a thin plate-shaped compression molded material was obtained. Subsequently, in the crushing step, the compression molded material was crushed, whereby a crushed material (formed granular material) was obtained. Thereafter, the formed granular material having passed through a sieve with a mesh size of 710 µm in the classification step was used as the granular composition (granule) of Example 6. At this time, the compression molded material was crushed such that all crushed material passed through the sieve. Incidentally, in the following Examples 7 and 8 and Comparative Example 6, as Compound (I), D-mannitol, and cornstarch, the same materials as those in Example 6 were used.

### Example 7

A granular composition of Example 7 was prepared using a tableting compression method. In the mixing step, 0.2 mg of Compound (I), 930 mg of D-mannitol, and 19.8 mg of cornstarch were placed in a fluidized bed device (MP-01, manufactured by Powrex Corporation), and a 10% hydroxypropyl cellulose (HPC-SSL, manufactured by Nippon Soda Co., Ltd.) aqueous solution was sprayed thereon while mixing the materials. By doing this, a granule (mixture) containing 50 mg of hydroxypropyl cellulose was obtained. In the obtained granule, 15 mg of magnesium stearate (magnesium stearate special product, manufactured by Taihei Chemical Industrial Co., Ltd.) was mixed, whereby 1015 mg of a mixture was obtained. Subsequently, in the compression molding step, compression molding was performed by applying a pressure of 780.9 N/mm² to the mixture using a rotary-type tableting machine (Correct, manufactured by Kikusui Seisakusho, Ltd.), whereby a plurality of disk-shaped granular compositions having a diameter of about 2 mm and a mass of 5 mg per granule were obtained as Example 7.

### Example 8

A granular composition of Example 8 was prepared using an extrusion granulation method. In the mixing step, 0.2 mg of Compound (I), 960 mg of D-mannitol, and 19.8 mg of cornstarch were placed in a stirring mixing granulator (VG-05, manufactured by Powrex Corporation), and a 10% hydroxypropyl cellulose aqueous solution was added thereto while mixing the materials. By doing this, 1000 mg of a kneaded material (mixture) containing 20 mg of hydroxypropyl cellulose was obtained. Incidentally, as hydroxypropyl cellulose, the same material as that in Example 7 was used.

The obtained kneaded material was extruded through a screen with a hole diameter of 0.5 mm using a wet-type extrusion granulator (MultiGran MG-55, manufactured by DALTON Corporation), whereby a granulated material (compression molded material) was formed. The obtained granulated material was dried at 60°C, and subsequently, in the crushing step, the granulated material was crushed, whereby a crushed material (formed granular material) was obtained. Thereafter, the formed granular material having passed through a sieve with a mesh size of 1700 µm in the classification step was used as the granular composition (granule) of Example 8. At this time, the compression molded material was crushed such that all crushed material passed through the sieve.

### Comparative Example 1

20mg of the mixture which did not undergo the compression molding step and the following steps in Example 1 was used as Comparative Example 1. The preparation was performed in the same manner as in Example 1 except this.

### Comparative Example 2

20mg of the mixture which did not undergo the compression molding step and the following steps in Example 2 was used as Comparative Example 2. The preparation was performed in the same manner as in Example 2 except this.

### Comparative Example 3

20mg of the mixture which did not undergo the compression molding step and the following steps in Example 3 was used as Comparative Example 3. The preparation was performed in the same manner as in Example 3 except this.

### Comparative Example 4

20mg of the mixture which did not undergo the compression molding step and the following steps in Example 4 was used as Comparative Example 4. The preparation was performed in the same manner as in Example 4 except this.

### Comparative Example 5

20mg of the mixture which did not undergo the compression molding step and the following steps in Example 5 was used as Comparative Example 5. The preparation was performed in the same manner as in Example 5 except this.

### Comparative Example 6

The mixture which did not undergo the compression molding step and the following steps in Example 7 was used as Comparative Example 6. The preparation was performed in the same manner as in Example 7 except this.

### Comparative Example 7

As Comparative Example 7, only Compound (I) was compression molded in the same manner as in Example 1 without using the excipient. The preparation was performed in the same manner as in Example 1 except this.

### Comparative Example 8

Compound (I) which did not undergo compression molding was used as Comparative Example 8. The preparation was performed in the same manner as in Comparative Example 7 except this.

With respect to the granular compositions of Examples 1 to 8, and Comparative Examples 1 to 8 prepared as described above, a dissolution test was performed. The dissolution test was performed according to the dissolution test method of the Japanese Pharmacopoeia, Seventeenth Edition. By using a dissolution testing device (NTR-6000 series, manufactured by Toyama Sangyo Co., Ltd.), the dissolution test was performed by a paddle method using water as a dissolution test liquid. At this time, the volume of the dissolution test liquid was set to 900 mL, the temperature of the dissolution test liquid was set to 37 ± 0.5°C, and the paddle rotation rate was set to 50 rpm. With respect to the respective Examples and the respective Comparative Examples, the total amount was added to the dissolution test liquid, and the dissolution test liquid was sampled at 5, 10, 15, 30, 45, 60, 90, and 120 minutes after the start of the test, and filtered through a 0.45-µm filter (manufactured by Whatman GE Healthcare Biosciences), and then, the dissolution rate of Compound (I) was measured using high performance liquid chromatography.

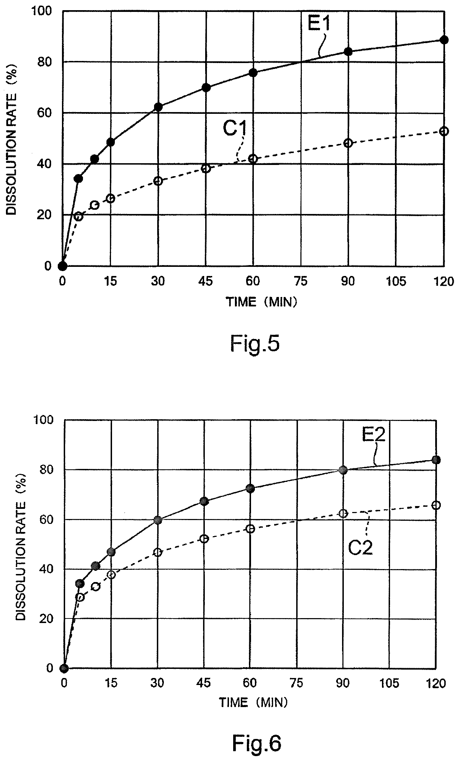

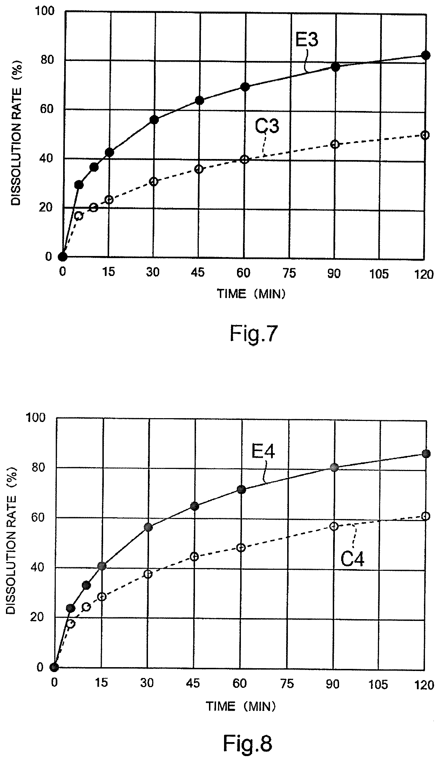

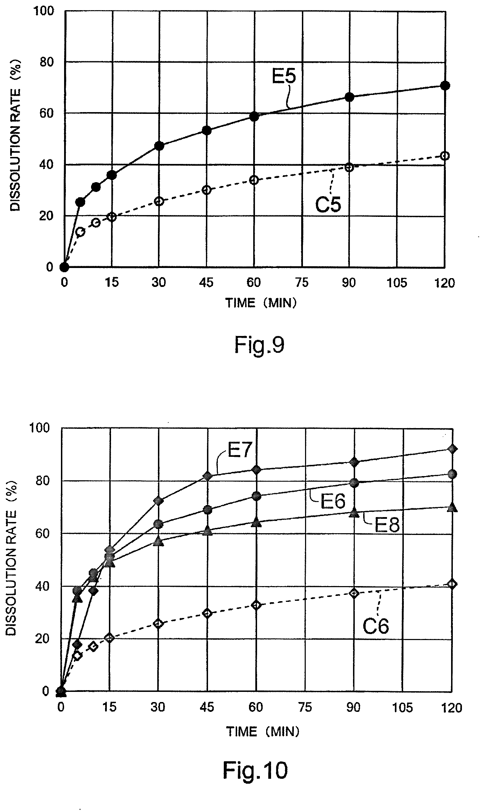

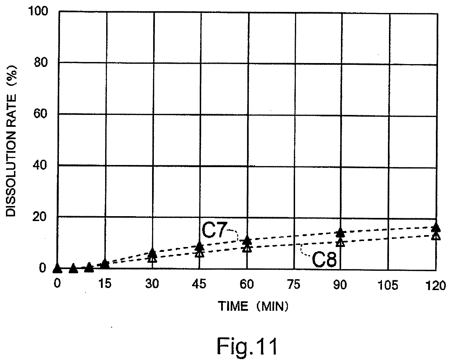

FIGS. 5 to 9 show the time course of the dissolution rate of Compound (I) in the granular compositions of Examples 1 to 5, respectively, and also show the time course of the dissolution rate of Compound (I) in Comparative Examples 1 to 5, respectively. FIG. 10 shows the time course of the dissolution rate of Compound (I) in the granular compositions of Examples 6 to 8 and Comparative Example 6. FIG. 11 shows the time course of the dissolution rate of Compound (I) in Comparative Examples 7 and 8. In FIGS. 5 to 11, the vertical axis represents the dissolution rate (unit: %) and the horizontal axis represents a time (unit: min). The solid lines E1 to E8 represent the cases of Examples 1 to 8, respectively, and the broken lines C1 to C8 represent the cases of Comparative Examples 1 to 8, respectively.

As shown in FIGS. 5 to 9, the granular compositions of Examples 1 to 5 improved the dissolution rate of Compound (I) as compared with Comparative Examples 1 to 5. Accordingly, it is found that the dissolution property of Compound (I) is improved by compression molding the mixture of Compound (I) and the excipient.

As shown in FIG. 10, the granular compositions of Examples 6 to 8 improved the dissolution rate of Compound (I) as compared with Comparative Example 6. Further, in each of the granular compositions of Examples 6 to 8, the dissolution rate of Compound (I) at 120 minutes after the start of the test was 70% or more. On the other hand, in Comparative Example 6 which did not undergo the compression molding step, the dissolution rate of Compound (I) at 120 minutes after the start of the test was 41.2%. Accordingly, it is found that the dissolution property of Compound (I) can be improved even if the compression molding step is performed by a roller compression method, a tableting compression method, or an extrusion granulation method.

As shown in FIG. 11, the dissolution rates of Comparative Examples 7 and 8 were less than 20% at 120 minutes after the start of the test, and there was no significant difference between the dissolution rates of Comparative Examples 7 and 8. Accordingly, it is found that an excipient selected from the group consisting of a sugar alcohol, a starch, and a saccharide is necessary for improving the dissolution property of Compound (I) in the granular composition.

Incidentally, the dissolution rate in a case where each of acetaminophen, indomethacin, and celiprolol hydrochloride was mixed with an excipient and then, the resulting mixture was compression molded in the same manner as in this embodiment was substantially equivalent to the dissolution rate in a case where the mixture was not compression molded.

A detailed mechanism for the improvement of the dissolution property of Compound (I) in the granular composition by compression molding the mixture of Compound (I) and the excipient is not clear but is presumed that an interaction occurred between Compound (I) and the excipient by the compression molding step. Incidentally, the present invention is not restricted to the above-mentioned mechanism.

Subsequently, an experiment for examining the relationship between the porosity of the granular composition and the dissolution property of Compound (I) was performed. In the mixing step, 0.2 mg of Compound (I), D-mannitol, cornstarch, low-substituted hydroxypropyl cellulose, hydroxypropyl cellulose, and magnesium stearate were mixed, whereby a mixture was obtained. Incidentally, as Compound (I), D-mannitol, cornstarch, low-substituted hydroxypropyl cellulose, hydroxypropyl cellulose, and magnesium stearate, the same materials as used in the above-mentioned Examples were used.

In the compression molding step, a pressure was applied to the mixture using a tableting compression method, whereby a disk-shaped granule was formed. This granule was determined to be a granular composition used in this experiment. At this time, the pressure applied to the mixture was made variable within a range of 0 to 509.6 N/mm².

Subsequently, the mass M (unit: g) per granular composition was measured, and also the volume V (unit: mm³) per granular composition was calculated based on the diameter and the thickness of the granular composition. Here, the volume V is an apparent volume including voids. Further, by using a dry-type automatic densimeter (AccuPyc II 1340, manufactured by Shimadzu Corporation), the true density ρ (unit: g/mm³) of the mixture itself (the granular composition including no voids) in the granular composition was measured by a fixed volume expansion method. Then, the porosity ε (unit: %) of the granular composition was calculated according to the following formula (1).$ε = 100 \times \left(V-M/ρ\right) / V$ (1)

After the porosity ε was calculated, with respect to the granular composition, a dissolution test was performed in the same manner as the above-mentioned dissolution test.

As a result of this experiment, when the pressure applied to the mixture in the compression molding step was large, the porosity ε was small, and the dissolution rate of Compound (I) was large. Then, it was found that when the porosity ε of the granular composition is 45% or less, the dissolution rate of Compound (I) is sufficiently larger than the dissolution rate in a case where the compression molding step is not performed.

Incidentally, in this experiment, the volume V (apparent volume) of the granular composition was calculated based on the diameter and the thickness and the porosity ε was determined. However, for example, the porosity ε may be determined using a tap density measuring method. Specifically, a weighed sample (a plurality of granular compositions) is placed, for example, in a measuring cylinder or the like, and thereafter, the measuring cylinder is lightly tapped until the degree of bulk reduction becomes 0, so that gaps between respective granular compositions in the sample are reduced. Then, the volume V (apparent volume) of the sample is measured by reading the scale of the measuring cylinder. Thereafter, the true density ρ of the sample is measured using a dry-type automatic densimeter, and the porosity ε is determined from the above formula (1) . According to this method, it is also possible to easily determine the porosity ε of the granular composition having an irregular shape.

### Industrial Applicability

The present invention can be utilized for a granular composition containing Compound (I) and an excipient.

## Claims

1. A production method for a granular composition containing the following Compound (I), comprising a step of compression molding a mixture obtained by mixing the Compound (I) and at least one or more excipients selected from the group consisting of a sugar alcohol, a starch, and a saccharide, thereby obtaining a compression molded material:

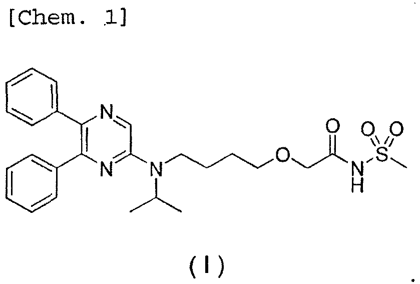

2. The production method for a granular composition according to claim 1, wherein the dissolution property of the Compound (I) in the granular composition is higher than the dissolution property of the Compound (I) in the mixture before the compression molding step.

3. The production method for a granular composition according to claim 1 or 2, wherein the porosity of the granular composition is 45% or less.

4. The production method for a granular composition according to any one of claims 1 to 3, wherein the granule size of the granular composition is smaller than 5 mm.

5. The production method for a granular composition according to any one of claims 1 to 4, wherein the compression molding step is performed by any one of a roller compression method, a tableting compression method, a briquetting method, a slugging method, and an extrusion granulation method.

6. The production method for a granular composition according to claim 5, wherein in the compression molding step,
the extrusion granulation method is performed using an extruder which extrudes the mixture through a hole portion, and
the diameter of the hole portion is from 0.2 mm to 0.5 mm.

7. The production method for a granular composition according to any one of claims 1 to 6, wherein a crushing step of crushing the compression molded material is further included.

8. The production method for a granular composition according to any one of claims 1 to 7, wherein the granular composition is a granule, a powder, a filler of a capsule, a granular tablet, a dry syrup, or a fine granule.

9. A dissolution property improvement method for improving the dissolution property of the following Compound (I) in a granular composition containing the Compound (I), comprising a step of compression molding a mixture obtained by mixing the Compound (I) and at least one or more excipients selected from the group consisting of a sugar alcohol, a starch, and a saccharide, thereby obtaining a compression molded material:

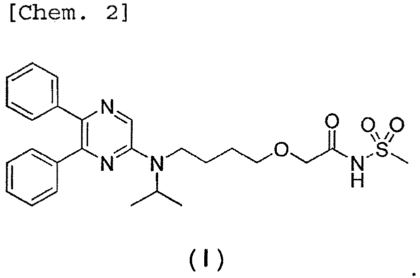

10. The dissolution property improvement method according to claim 9, wherein the dissolution property of the Compound (I) in the granular composition is higher than the dissolution property of the Compound (I) in the mixture before the compression molding step.

11. The dissolution property improvement method according to claim 9 or 10, wherein the porosity of the granular composition is 45% or less.

12. The dissolution property improvement method according to any one of claims 9 to 11, wherein the granule size of the granular composition is smaller than 5 mm.

13. The dissolution property improvement method according to any one of claims 9 to 12, wherein the compression molding step is performed by any one of a roller compression method, a tableting compression method, a briquetting method, a slugging method, and an extrusion granulation method.

14. The dissolution property improvement method according to claim 13, wherein in the compression molding step,
the extrusion granulation method is performed using an extruder which extrudes the mixture through a hole portion, and
the diameter of the hole portion is from 0.2 mm to 0.5 mm.

15. The dissolution property improvement method according to any one of claims 9 to 14, wherein a crushing step of crushing the compression molded material is further included.

16. The dissolution property improvement method according to any one of claims 9 to 15, wherein the granular composition is a granule, a powder, a filler of a capsule, a granular tablet, a dry syrup, or a fine granule.

17. A granular composition which is in a state where the following Compound (I) and at least one or more excipients selected from the group consisting of a sugar alcohol, a starch, and a saccharide are mixed, and has a porosity of 45% or less:

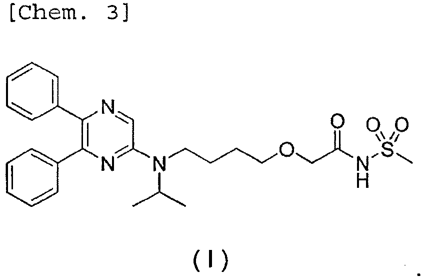

18. The granular composition according to claim 17, wherein the granular composition has a granule size smaller than 5 mm.
